# EUROPÄISCHE PATENTANMELDUNG

(11) **EP 1 780 248 A2**
(43) Veröffentlichungstag der Anmeldung: **02.05.2007**
(21) Anmeldenummer: 06018066.8
(22) Anmeldetag: 30.08.2006
(51) Int. Cl.: C09D 101/18, C08J 3/20, A61Q 3/02

(54) **Verfahren und Vorrichtung zu Herstellung eines Lackrohstoffes**

(30) Priorität: 07.10.2005 DE 102005048040
(71) Anmelder: Hagedorn AG, 49078 Osnabrück (DE)
(72) Erfinder: Mons, Horst Dr., 49477 Ibbenbüren (DE); Lingemann, Enno, 49088 Osnabrück (DE)
(74) Vertreter: Bünemann, Egon

(57) **Zusammenfassung**

Bei einem Verfahren zur Herstellung eines insbesondere Cellulosenitrat als thermoplastisches Bindemittel enthaltenden Rohstoffes für Lacke, Farben o. dgl. Produkte ist vorgesehen, daß in diesem Rohstoff unter Zusatz von Lösungsmittel und Weichmacher auch ein eine plättchenförmige Struktur aufweisender Zuschlagstoff verteilt wird. Erfindungsgemäß wird das thermoplastische Bindemittel unter Zusatz von Lösungsmittel und Weichmacher oder ohne diesen zu einem niedrigviskosen Teig auf 40°C bis 70°C aufgeheizt und dabei direkt mit dem plättchenförmigen Zuschlagstoff gemischt. Bei weiterem Lösungsmittelzusatz wird diese endproduktnahe Mischung in eine bei 60°C bis 110°C plastifizierte teigige Konsistenz überführt und als hoch- oder niedrigviskose Mischung geknetet, derart, daß nach Ablauf einer definierte Scherkräfte in die Mischung eintragenden Knetphase ein den Zuschlagstoff in dispergierter Form enthaltender Lackrohstoff bereitgestellt wird.

## Beschreibung

Die Erfindung betrifft ein Verfahren und eine Vorrichtung zur Herstellung eines insbesondere Cellulosenitrat als thermoplastisches Bindemittel enthaltenden Rohstoffes zur Lackherstellung gemäß dem Oberbegriff des Anspruchs 1, 13 bzw. 19.

Bei bekannten Verfahren zur Herstellung von Lackrohstoffen wird in ein thermoplastisches Bindemittel ein eine plättchenförmige Struktur aufweisender Zuschlagstoff eingearbeitet, um danach ein Endprodukt mit entsprechenden optischen Effekten zu erreichen. In einer Veröffentlichung gemäß "Polymers and Thickeners in Nail-Care-Products" (Journals "Cosmetics & Toiletries" pp. 70-82, Vol. 103 Dec. 1988, /1/) wird ausgehend von einem Lackrohstoff die Herstellung eines Nagellacks beschrieben. In diesem wird Cellulosenitrat als Bindemittel trotz bekannter Sicherheitsrisiken verarbeitet. Denn das Cellulosenitrat bewirkt eine sehr gute Pigmentbenetzung, rasche physikalische Trocknung und bessere Beschichtungseigenschaften, so daß das Cellulosenitrat als der ,primary film former im Nagellacksektor angesehen wird. Bevorzugt werden hier als Lösungsmittel Ethanol und Ethylacetat, als bedeutender aktueller Weichmacher ATBC (Acetyltributylcitrat) und organisch modifizierte Schichtsilikate als rheologisches Additiv genannt. Diese Schichtsilikate werden in vordispergierter Form als Gelkonzentrate eingesetzt. Dieses Zwischenprodukt wird sehr zeit- und arbeitsaufwendig hergestellt und durch mechanische und chemische Einwirkung wird ein Produkt erzielt, das weiterhin unzerteilte Plättchenstapel enthält und deshalb nicht vollständig das eigentliche Potential seiner rheologischen (Thixotropie) und koloristischen Wirksamkeit (Brillanz, Glanz, Transparenz) entfalten kann.

In den Patentschriften DE 28 54 109 C3 /2/ und US 4,229, 227 /3/ der Shiseido Co. Ltd. werden Verfahren zur Herstellung einer teilchenförmigen Chipmasse als Precursormaterial für die Nagellackherstellung beschrieben. Ein Gemisch aus 47-Gew.% organisch modifiziertem Montmorrillionit, 41-Gew.% Nitrocellulose und 12-Gew.% Polyethylenglykol wurde auf einem Zweistufen-Walzenmischer unter Erhitzen und Komprimieren vermahlen. Die Oberflächentemperatur der ersten Walze wurde hierbei mit 40°C und die der zweiten Walze auf 60°C eingestellt. Mit geringfügig veränderter Zusammensetzung wurden in anderen Chipmassen-Ansätzen jeweils der eingesetzte Nitrocellulose-Typ durch eine höherviskosere Nitrocellulose und der Weichmacher durch Diethyladipat bzw. Dibutylphthalat ersetzt. Mit 18-Gew.% dieser Chipmassen wurden durch Lösen in einem Lösemittelgemisch Nagellacke hergestellt. Dieser wird mit einem Standardnagellack verglichen, der lediglich durch einfaches Vermischen der Ausgangs-Komponenten der Chipmassen und Lösen in gleichen Lösemittelgemisch hergestellt ist. Es ergeben sich hinsichtlich Glanz und Transparenz für das nur durch Walzbearbeitung geformte Chip-Material deutliche Vorteile gegenüber dem herkömmlichen Herstellverfahren. In den Formulierungen abgeleiteter pigmentierter Nagellacke ist eine deutliche Verringerung der Sedimentierung der enthaltenen Pigmente nachweisbar, die wiederum direkt auf die Erhöhung der thixotropen Eigenschaften des Montmorrillionit-Schichtsilikates durch die Walzenbehandlung zurückgeführt werden. Festzustellen ist jedoch, daß die beschriebenen teilchenförmigen Chipmassen dennoch nicht ausreichend dispergiert sind. Der in den Nagellackrezepturen für eine ausreichende thixotrope Wirksamkeit gewählte, vergleichsweise sehr hohe Anteil von mehr als 8-Gew.% an Montmorrillionit-Material weist deutlich auf diesen Nachteil hin. Diese hohe Additiv-Konzentration wirkt darüber hinaus nachteilig auf die nur noch eingeschränkt möglichen Rezeptierungsmengen der anderen Festkörperkomponenten des Lackes und führt damit zu schlechten Applikations- und Gebrauchseigenschaften des Nagellackes.

Bei einem Verfahren zur Herstellung einer Perlglanzpigment-Zubereitung gemäß DE 196 55 285 B4 /4/ wird ein Perlglanzpigment mit einem Bindemittel angepastet und danach durch Pressen, Verdichten, Extrudieren oder Pelletieren in eine kompakte Teilchenform gebracht. Die Bearbeitung des Zwischenprodukts erfolgt dabei in Pastenform und für die nachfolgende Konfektionierung des Lackrohstoffes sind an sich bekannte Verfahrensschritte durch Tablettieren, Brikettieren, Pelletieren, Granulieren oder Sprühgranulieren vorgesehen, um aus der pastigen Konsistenz entsprechende Preßteile als Lackrohstoff zu erzeugen. Nach diesem Formen des Produktes wird zur Erzeugung einer mechanischen Widerstandsfähigkeit ein zusätzlicher Trocknungsvorgang durchgeführt. Dabei führen die allgemein bekannten und zur Dispergierung von Zuschlagstoffen eingesetzten Aggregaten (z. B. Perlmühlen, Sandmühlen, Mischer, Walzenstühle, hochtourige Rühraggregate) nur zu einer unzureichenden Zerteilung in Plättchen oder durch zu hohe mechanische Mahlbelastung zu einem unerwünschten Bruchanteil, so daß die angestrebten Effekte im Lackrohstoff bzw. dem daraus formulierten Endprodukt nachteilig beeinträchtigt sind.

Bei der Dispergierung von Perlglanzpigmenten ist zu beachten, daß insbesondere die effektgebende dünne Beschichtung auf dem Plättchensubstrat durch die mechanische Beanspruchung, z. B. durch das Mahlgut in Perlmühlen, nicht beschädigt wird. Am Endprodukt wird sonst dadurch die Farbgebung beeinträchtigt, die entscheidende planparallele Ausrichtung von Pigmentplättchen in der Beschichtung ist durch die erzeugten Bruchstücke erschwert und damit wird auch die optimale Glanzausbildung, Brillanz und Transparenz eingeschränkt.

Bei Anwendung von organisch modifizierten Montmorrillionit-Tonen wurde festgestellt, daß einerseits die Zerteilung der Plättchenstapel in Einzelplättchen nur unzureichend erfolgt und andererseits durch Erzeugung eines nicht unerheblichen Trümmeranteils der Aufbau eines gewünschten Thixotropie-Effektes geschwächt ist. Im Anschluß an den eigentlichen Dispergierprozeß entsteht nach einer weiteren Verarbeitungsabfolge, die eine sogenannte Aktivierung und eine Reifezeit beinhaltet, das für die Nagellackherstellung übliche, pastenförmige Gelkonzentrat. Dieses Gel enthält in der Regel leichtflüchtige, brennbare, organische Lösemittel, die während der Bereitstellungszeit verdampfen könnten und entsprechend aufwendige Vorkehrungen beim Gel-Herstellungsprozeß und zu dessen Zwischenlagerung erfordern.

Bei der im Stand der Technik angestrebten Thixotropie handelt es sich phänomenologisch um ein reversibles Viskositätsverhalten, bei dem zunächst eine bestimmte höhere Viskosität in einer unbewegten Lösung vorliegt. Wird diese gerührt, sinkt die Viskosität abrupt ab und steigt sofort wieder auf das alte Niveau, sobald die Bewegung eingestellt wird. Dieses Verhalten wird einerseits ausgenutzt, um eine nahezu spritz- und tropfenfreie Applikation zu erzielen und andererseits das Absetzen oder Sedimentieren von schweren Rezepturkomponenten (z. B. Perlglanzpigmenten, TiO2 etc.) insbesondere während der Lagerung zu verhindern.

Die Erfindung befaßt sich mit dem Problem, ein Verfahren und eine Vorrichtung zur Herstellung eines Lackrohstoffes zu schaffen, womit bei hoher Produktivität und Wirksamkeit sowie verringertem Einsatz von plättchenförmigem Zuschlagstoff ein Lackrohstoff erreichbar ist, der neben einer optimalen Lagerform eine deutliche Qualitätsverbesserung der rheologischen und koloristischen Eigenschaften für eine Vielzahl von Endprodukten ermöglicht.

Die Erfindung löst diese Aufgabe durch ein Verfahren und eine Vorrichtung mit den Merkmalen des Anspruchs 1, 13 bzw. 19. Weitere vorteilhafte Ausgestaltungen ergeben sich aus den Ansprüchen 2 bis 12, 14 bis 18 sowie 20 bis 22.

Mit dem erfindungsgemäßen Verfahren und einer dafür vorgesehenen Vorrichtung gelingt eine besonders effiziente Bearbeitung und Bereitstellung von Lackrohstoff-Mischungen, die Cellulosenitrat (NC) als Bindemittel und zumindest einen Zuschlagstoff enthalten. Es wurde erkannt, daß die als Zwischenprodukte mit dem ein hohes Gefährdungspotential verursachenden Cellulosenitrat (NC) zu verarbeitenden Komponenten in einer eine entsprechend dem gewählten Zuschlagstoff variable Viskosität aufweisenden teigigen Konsistenz auch geknetet werden können, so daß durch einen angemessenen Scherkrafteintrag eine optimale Verteilung und Dispergierung von Hektoriten, Perlglanzpigmenten o. dgl. plättchenförmigen Zuschlagstoffen in dem NC-Lackrohstoff erreicht wird.

Bei dem in seinen Mischphasen und Bearbeitungsbedingungen gesteuert ablaufenden Knetvorgang werden definierte, entsprechend dem Zuschlagstoff wählbare Scherkräfte in das Zwischenprodukt eingebracht. Festgestellt wurde, daß in der NC-Lackrohstoff-Mischung überraschenderweise die einzelnen Plättchen eines zugeführten Effektpigmentes auch bei hohem Gewichtsanteil von mehr als 30 % - vorzugsweise bis 80 Gew.-% - vollständiger und schneller in der Teigmischung dispergiert werden. Diese verbesserte und schnellere Dispergierung ist auch bei mit einem Gewichtsanteil von 25 - 40 % - vorzugsweise mit 30 Gew.-% - eingesetzten feinteiligen Schichtsilikaten oder anderen plättchenförmigen Zuschlagstoffen nachweisbar. Im Vergleich zu bekannten Gel- oder Pastenpräparationen wird mit diesen erfindungsgemäßen Produkten zusätzlich zum geringeren Materialeinsatz auch eine wesentlich verkürzte Bearbeitung erreicht.

Diese bisher von der Fachwelt wegen hoher Explosionsgefahr abgelehnte Bearbeitung derartiger Cellulosenitrat enthaltender Mischungen in Vorrichtungen erfindungsgemäßer Ausführung kann überraschenderweise dadurch ermöglicht werden, daß eine entsprechende Zusammensetzung des Knetteiges auch bezüglich des erreichbaren Viskositätsniveaus in der Zusatzstoff-Konzentration ermittelt wurde und diese hinsichtlich des Gefährdungspotentials optimiert wird. Entgegen der naheliegenden Wahl (Stand der Technik gemäß /1/, /2/, /3/, /4/) einer höheren Zusatzstoff-Konzentration führen die erfindungsgemäßen Verfahrensbedingungen mit einer scheinbar unökonomisch niedrigen Konzentration von Hektorit bzw. einer besonders hohen Konzentration an Perlglanzpigment zu einem optimalen Dispergiererfolg, der in Ansehung bekannter Techniken so nicht zu erwarten war.

Mit der optimierten Knet-Bearbeitung des teigigen Zwischenprodukts als hochviskose Masse erfolgt ein die Feinverteilung bewirkender Schervorgang, so daß danach erreichte Hektoritpräparationen als konfektionierte Endprodukte überraschend verbesserte Anwendungseigenschaften aufweisen. Diese Wirksamkeit ist anhand von Vergleichsversuchen und entsprechenden rasterelektronenmikroskopischen Aufnahmen (REM) in der erzeugten Lack- bzw. Farbschicht nachweisbar. Nachweisbar ist dabei auch, daß der Oberflächenglanz mit erfindungsgemäßen Lackrohstoffen, insbesondere mit dispergiertem Hektorit als Zuschlagstoff, im direkten Vergleich mit entsprechend bekannten Produkten zu einer bis zu 10fachen Verbesserung der Vergleichswerte führt. Unerwartet ist, daß an jeweiligen einen um 50 % erhöhten Bentonit-Anteil aufweisende Farbschicht auf Basis des gekneteten Lackrohstoffes als dispergiertes Ausgangsmaterial überraschenderweise auch eine erkennbar höhere Glanzwirkung durch erhöhten Glanzgrad nachweisbar ist. Zusätzlich ergibt sich, daß durch eine optimale planparallele Ausrichtung der Einzelplättchen des Bentonits nicht nur die vorgenannte Glanzwirkung sondern in Folge höherer Konzentration auch eine höhere Kratzfestigkeit durch die sich näher an der Oberfläche der erzeugten Lackschicht planparallel ausrichtenden Zuschlagstoffe erreicht wird.

Bei Anwendung des erfindungsgemäßen Lackrohstoffes in der Nagellackherstellung werden auch zeit- und arbeitsintensive Zwischenschritte zur Herstellung des bisher üblichen Gelkonzentrates eingespart. Durch die abweichend von bekannten Gel-Produkten nunmehr trockene Festkörperkonzentrat-Rezeptur werden die enthaltenen Lackrohstoffe dennoch staubfrei und ohne Lösungsmittelemissionen in einer lagerstabilen Form zugänglich gemacht. Die bisher übliche Gelkonzentrat-Zwischenstufe kann entfallen, da der benötigte Hektorit bereits vordispergiert im NC-Lackrohstoff vorliegt und keiner zusätzlichen Aktivierung bei der Anwendung bedarf. Damit ist eine sofortige Anwendbarkeit möglich und für den Verbraucher eine wesentliche Zeiteinsparung erreichbar.

Die Wirksamkeit der in diesen erfindungsgemäßen Präparationen enthaltenen plättchenförmigen Substrate hängt in den jeweiligen Anwendungsbereichen insbesondere davon ab, ob diese als Zuschlagstoff eingebrachten Substanzen hinreichend feindispergiert sind und im Idealfall als Einzelplättchen vorliegen. Mit dem trotz NC-Bindemittel eingesetzten und steuerbaren Knetverfahren wird erreicht, daß bereits während des Dispergiervorgangs auch gleichzeitig eine im wesentlichen vollständige Benetzung der Teilchenoberflächen mit Bindemittelmolekülen erfolgt und damit eine Reagglomeration verhindert ist. Es ist bekannt, daß die ursprünglich vorliegenden Plättchenstapel eines Schichtsilikates, insbesondere des Hektorits, aus einer Vielzahl von Schichteinheiten mit einer Tafelstärke von ca. 1 nm bestehen. Diese sind interlamellar durch ionische van der Waals-Kräfte zusammengehalten. Bei dem erfindungsgemäß ablaufenden Knetvorgang gelingt es, diese Schichteinheiten weitgehend zerstörungsfrei zu Einzelplättchen zu dispergieren und simultan mit Bindemittelmolekülen zu stabilisieren, so daß zur Präparation von Nagellacken, Druckfarben, Lack-Aerosolen o. dgl. Endprodukte ein für diese ergiebigerer und qualitativ hochwertigerer Lackrohstoff bereitgestellt wird und damit die Endprodukte kostengünstiger herstellbar sind.

Bei Perlglanzpräparationen ist bisher nur bekannt, daß feinteilige Zusätze wie z. B. Diatomeenerde bei der Dispergierung beigemischt werden, um die Reagglomeration der Plättchen zu verhindern. Diese Zusätze wirken als Abstandhalter, sind aber gleichzeitig hinderlich bei der optimalen planparallelen Ausrichtung der Perlglanzpigmente und senken damit den maximalen erreichbaren koloristischen Effekt bei bekannten Präparationen. Mit der erfindungsgemäßen Verfahrensführung werden diese Nachteile überwunden.

Erfindungsgemäß wird der bekannte Effekt genutzt, daß durch Wasserstoffbrücken an den Plattenkanten dreidimensional miteinander verknüpfter Hektorit-Plättchen im Idealfall in einer Lackformulierung im Ruhezustand ein Gel aufgebaut werden kann. Diese Wasserstoffbrücken verhindern durch Einschluß in den sich ausbildenden Hektorit-Zellen eine Sedimentation und Agglomeration der darin enthaltenen, ebenfalls feinstdispergierten Pigmentteilchen. Diese Verknüpfungen im Lackrohstoff tragen entscheidend zur Stabilisierung der Brillanz, Transparenz und Glanzhaltung von damit herstellbaren Beschichtungen bei und gleichzeitig sind die vorstehend geschilderten, optimalen Eigenschaften dieser Farbpräparationen für einen langen Zeitraum, z. B. bei der Lagerung in trockenem Zustand, sichergestellt.

Durch das vorgeschlagene Herstellungsverfahren werden für das angestrebte veredelte Zwischenprodukt in Form des Lackrohstoffes geringere Mengen an Lösungsmitteln beim Endverarbeiter benötigt und durch eine vollständige Dispergierung zu Einzelplättchen mit Hektorit wird eine effizientere thixotrope Einstellung in dem daraus herstellbaren Nagellack erreicht. Bei vergleichbaren thixotropen Einstellungen von Rezepturen führen die erfindungsgemäßen Zusammensetzungen einerseits zu einer Materialeinsparung beim Rheologieadditiv und andererseits zu einer Verringerung unerwünschter Farbeinflüsse, beispielsweise eines agglomerisierten, gelblichbraun getönten Schichtsilikates. Gleichzeitig sind mit erfindungsgemäßen Rezepturen bei einem einzigen Aufstrich größere Schichtdicken auftragbar. Bei NC-Rezepturen mit Perlglanzpigmenten werden durch verbesserte, zerstörungsfreie Dispergierung ein optimaler Glanz, eine gute Transparenz und eine verbesserte Brillanz erreicht. Diese Vorteile und mögliche Materialeinsparungen führen bei geringem Einsatz von Effektpigmenten zu einer wesentlichen Kostensenkung.

Weitere Einzelheiten und Wirkungen ergeben sich aus der nachfolgenden Beschreibung in Verbindung mit der Zeichnung, in der ein Ausführungsbeispiel des erfindungsgemäßen Verfahrens und der Vorrichtung schematisch veranschaulicht sind. In der Zeichnung zeigen:
- Fig. 1: eine schematische Übersicht des erfindungsgemäßen Verfahrensablaufs bei der Herstellung des Lackrohstoffes,
- Fig. 2: eine Prinzipdarstellung einer Vorrichtung zur Durchführung des Verfahrens zur Herstellung des Lackrohstoffes,
- Fig. 3 und Fig. 4: jeweilige Meßkurven des Viskositätsverhaltens von mit dem erfindungsgemäßen Lackrohstoff hergestellten Lacklösungen im Vergleich mit bekannten Lacklösungen,
- Fig. 5: ein Vergleichsbild mit Säulendiagrammen zum Glanzverhalten von Lackschichten bekannter Zusammensetzung und Lackschichten unter Verwendung des erfindungsgemäßen Lackrohstoffes,
- Fig. 6: eine Schnittdarstellung einer Lackschicht in REM-Aufnahme bei Anwendung eines Lackrohstoffes bekannter Zusammensetzung, und
- Fig. 7: eine Schnittdarstellung ähnlich Fig. 6 bei Anwendung eines den erfindungsgemäßen Lackrohstoff enthaltenden Lackes.

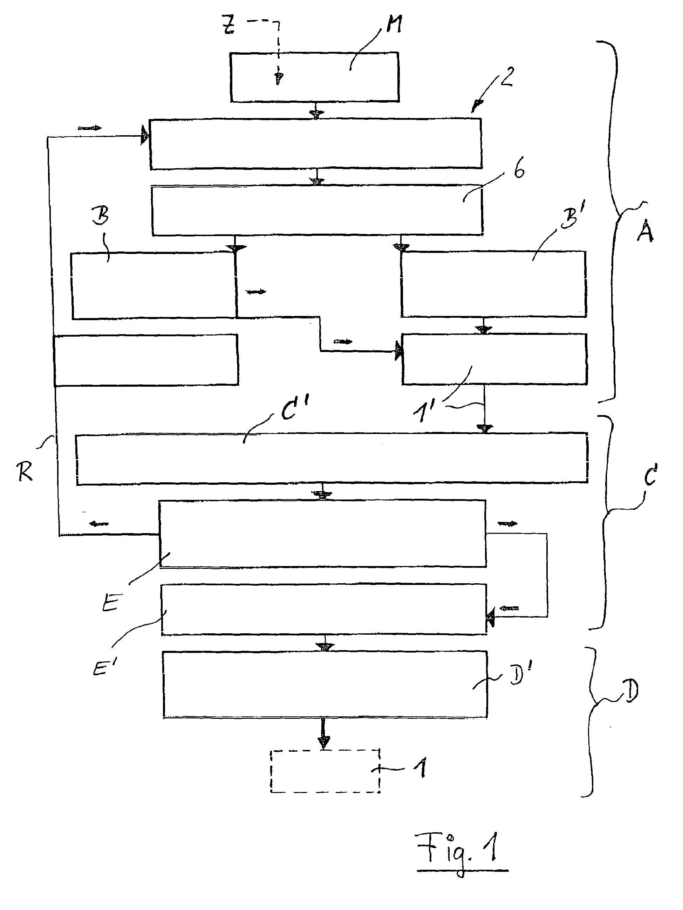

In Fig. 1 ist ein Verfahrensschema zur Herstellung eines am Ende einer Bearbeitungsstrecke als trockene Präparation entnehmbaren NC-Lackrohstoffes 1 dargestellt, der sich wie folgt zusammensetzt:
- 1 - 85 Gew.% eines in plättchenförmigem Aggregatzustand, natürlich oder synthetisch, vorliegenden Substrates (z. B. Montmorillionit, Smekrit, Hektorit, Phlogopit, Muskovit, aber auch organisch modifizierte Substrate, wie z. B. Bentonit®, Tixogel®, oder plättchenförmige Effektpigmente)
- 1 - 40 Gew.% eines der, dem Fachmann aus der Anwendung in Druckfarben, Lacken und Kosmetik bekannten, sogenannten Weichmachers (z. B. Acetyltriethylcitrat (ATC), Acetylbutylcitrat (ATBC), Phtalate, Sebacate, Adipate, Organophosphate, etc.) und
- 1 - 70 Gew.% Cellulosenitrat (NC) oder andere gebräuchliche, plastifizierbare Bindemittel (z. B. Acrylate, Polyurethane, etc.).

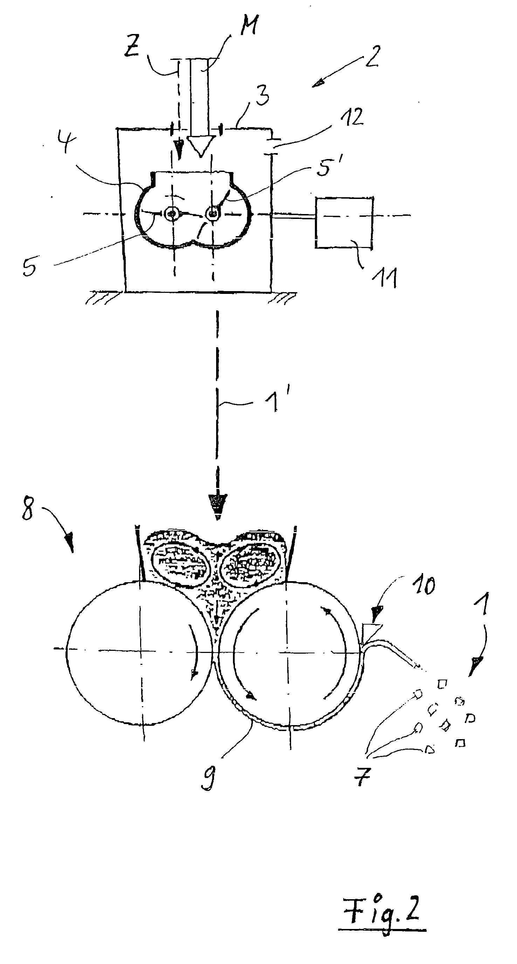

Die Verarbeitung dieser Cellulosenitrat (NC) enthaltenden Mischungen M erfolgt in einer eine Verteilphase A vorgebenden Verteil-Vorrichtung (Fig. 1), die insbesondere in Form eines diskontinuierlich arbeitenden Knet- Misch-Aggregates 2 (Fig. 2) ausgebildet ist. Dieses Aggregat 2 weist einen steuerbaren Antrieb 11 auf, so daß variierbare Scherkräfte in die entsprechende Rezepturkomponenten aufweisende Mischung M einbringbar sind.

Bevorzugt werden doppelarmige Kneter verwendet, die mit oder ohne Austragsschnecke einen Leistungseintrag von beispielsweise 0,1 bis 0,8 KW/kg mittels des Antriebs 11 ermöglichen. Das dargestellte Knet-Misch-Aggregat 2 ist in Form eines Trogkneters 3 vorgesehen, der im Knetwerk 4 zwei im Verhältnis 1:2 gegenläufige Knetarme 5, 5' als auf die Mischung einwirkende Baugruppe aufweist. In diesem auf einen definierten Scherkrafteintrag einstellbaren Aggregat 2 ist in der Verteilphase A eine Viskositätskontrolle 6 (Fig. 1) möglich. Bei hoher Viskosität erfolgt eine entsprechende Lösungsmittelzugabe B und bei zu niedriger Viskosität ein Entzug B' des Lösungsmittels. Das für die Verarbeitung der NC-haltigen Mischung M überraschend gut geeignete Knet-Misch-Aggregat 2 ist beheizbar und kühlbar, wobei über Öffnungen 12 das Lösungsmittel absaugbar ist und das Zwischenprodukt 1' einer Preßphase C oder direkt einer Endbearbeitungsphase D (Konfektionierung) zuführbar ist.

Das zur Aufnahme der Mischkomponenten vorgesehene Knet-Misch-Aggregat 2 wird zu Beginn auf 40 bis 70°C vorgeheizt. Als zugeführte Bindemittel kommen jeweilige Stoffe in Betracht, die in Druck- und Lackfarben Verwendung finden. Insbesondere sind dies thermoplastische Bindemittel, die bei Raumtemperatur fest sind und sich durch Zugabe von Wasser oder Lösungsmittel bei erhöhter Temperatur gelieren lassen. Besonders geeignet sind hoch-, niedrig- und mittelviskoses Cellulosenitrat (NC), Celluloseacetopropionate, Celluloseacetobutyrate, Polyvinylbutyrate, Polyvinylchlorid-Mischpolymerisate, Acrylate, Polyester und Ethylcellulosen. Eingesetzt werden diese Bindemittel sowohl einzeln als auch in Mischungen von zwei oder mehreren dieser Stoffe.

Als weitere Komponenten werden dem Ausgangsgemisch ein oder mehrere Weichmacher wie z. B. Acetlytriethylcitrat (ATC), Acetyltributylcitrat (ATBC), Phtalate, Sebacate, Adipate, Phosphate o. dgl. zugesetzt. Dieses Gemisch wird im Verlauf des Füllvorgangs auf ca. 60° bis 110°C aufgeheizt und dabei weiterhin Lösungsmittel zugefügt. Als Lösungsmittel kommen alle Flüssigkeiten in Betracht, die in der Lage sind, das Bindemittel zu benetzen und es anzulösen. Insbesondere eignen sich hierfür Ester (Methylacetat, Ethylacetat, Isopropylacetat, -..), Ketone (Aceton, MEK, MIBK), Alkohole (Ethanol, Propanol, Butanol, ...), aber zusätzlich auch Wasser.

Die Menge des Lösungsmittels richtet sich nach der jeweiligen Temperatur der Mischung M, dem Bindemittel, der Art des zugegebenen natürlich oder synthetisch vorliegenden Zuschlagstoffes Z in Plättchenform, dessen Anteils an der Gesamtrezeptur und der vorgesehenen Knetviskosität. Nach erfolgter Plastifizierung des Bindemittels im Knet-Misch-Aggregat 2 wird der zu dispergierende Zuschlagstoff Z zugegeben und anschließend der Mischung M so lange weiteres Lösungsmittel zugesetzt, bis ein bei 60°C bis 110°C homogener Teig entsteht. In dieser Phase können dispergiererleichternde Substanzen hinzugefügt werden, die (z. B. bei einer Dispergierung von Bentonit) die Ausbildung der Wasserstoffbrückenbindungen bzw. das Aufquellen von "Substratstapeln" beschleunigen. Die in Abhängigkeit vom Zuschlagstoff Z hoch- oder niedrigviskose Mischung M wird unter ständiger Viskositätskontrolle 6 geknetet.

Dieser permanent kontrollierbare Knetvorgang wird nach einer entsprechend der Mischung M variablen Bearbeitungszeit von ca. 0,5 bis 5 Stunden beendet und das den Zuschlagstoff Z in vordispergierter Form enthaltende Produkt kann direkt als Lackrohstoff 1 zur Weiterverarbeitung bereitgestellt werden. Zu einer praktischen Konfektionierung des Zwischenproduktes 1' sind in den Phasen C und D (Fig. 1) nachfolgende Verarbeitungsschritte vorgesehen, wobei diese allein oder auch in Kombination miteinander angewendet werden können.

Mit Hilfe einer Scherwalze (Phase C') kann das Zwischenprodukt 1' unter Einwirkung hoher Scherkräfte zu enddispergierten und pelletierten Strukturteilen 7 in Lieferform (z. B. Druckmesser ca. 2 bis 6 mm, Stärke 2 bis 4 mm) verarbeitet werden. Der damit erreichte Lackrohstoff 1wird als lieferfähiges Produkt über eine Trocknung (z. B. Bandtrocknung, Vakuumtrocknung, Trockenschrank) bei ca. 60 bis 80°C auf eine Restfeuchte von beispielsweise ca. 2 % eingestellt. Der damit erreichte Lackrohstoff 1 als zur Weiterverarbeitung bereitgestelltes Endprodukt zeigt zwar bei der nachfolgenden Anwendung einen höheren Viskositätsaufbau, weist aber gleichzeitig eine vergleichsweise schlechte Löslichkeit auf.

Das Zwischenprodukt 1' kann in der Phase C' auch über eine Doppelwalze 8 (Fig. 2) mit glatten Walzenoberflächen zu einem Fell 9 mit gleichmäßiger Dicke verarbeitet werden, so daß danach mit einer anschließenden Zerkleinerung (Phase D') in einer Hackanlagen 10 jeweilige Strukturteile 7 mit unregelmäßigem, plättchenförmigem Erscheinungsbild (z. B. mittlere Plättchendurchmesser ca. 0,5 cm, Plättchenstärke 1 bis 2 mm) gebildet werden. An die Bearbeitung in der Phase C' kann sich auch noch eine Dispergierkontrolle E mit zusätzlicher Trocknung E' anschließen, wobei diese Kontrolle E eine Rückkopplung R zum Aggregat 2 aufweist und damit eine Prozeßoptimierung bewirkt ist. Diese Glattwalz-Phase bei C' führt zu einem Lackrohstoff 1, der bei der Anwendung einen vergleichsweise schlechteren Viskositätsaufbau aufweist, gleichzeitig aber, da die Mischung beim Glattwalzen nicht so stark verdichtet wurde, in den Löslichkeits-Eigenschaften verbessert ist.

An Stelle der vorbeschriebenen Bearbeitung bei C' durch Walzenkonfigurationen kann zur Erlangung einer optimaleren Lieferform, unter gleichzeitiger Einwirkung von weiteren Scherkräften, auch eine Verarbeitung des Zwischenproduktes 1' durch Granulatoren (nicht dargestellt) erfolgen. Damit werden Strukturteile 7 mit Abmessungen von 0,3 cm Durchmesser und 0,3 cm Länge erreicht, so daß der so zerkleinerte Lackrohstoff 1 aufgrund einer großen Teilchenoberfläche eine gute Löslichkeit aufweist.

Ebenso ist denkbar, daß das Zwischenprodukt 1' mehrfach geknetet wird (Rückkopplung R). Die daraus resultierenden Strukturteile 7 des Lackrohstoffes 1 führen bei der Anwendung zwar zu einem deutlich schlechteren Glanz und geringerem Viskositätsaufbau, im Vergleich mit nach bisherigem Stand der Technik dispergierten Produkten sind aber auch die Eigenschaften dieses mehrfach gekneteten Lackrohstoffes 1 deutlich besser.

Bei umfänglichen Versuchen hat es sich gezeigt, daß das verwendete Knet-Misch-Aggregat 2 durch den Eintrag von Scherenergie geradezu ideal für die Dispergierung von plättchenartigen Substanzen Z geeignet ist. Diese Substanzklasse umfaßt die bekannten Perlglanzpigmente, Metallbronzepigmente, aber auch die bekannten Schichtsilikate in Form von Bentonit und Hektorit. Diese werden z. B. als keramische Füllstoffe zur physikalischen Verstärkung von Hochleistungswerkstoffen eingesetzt, aber insbesondere auch als Rheologie-Additive in wasserverdünnbaren oder lösemittelhaltigen Druckfarben und Lacken. Eine Sonderform dieser erfindungsgemäß verwendeten Zuschlagstoffe Z sind die Bentonite in Form eines Hektorits. Dieses ist ein Nanoplättchensubstrat, das bei der Verarbeitung besonders hohe Ansprüche an ein schonendes Dispergierverfahren stellt, so daß das Hektorit insbesondere zur Herstellung von Nagellacken eingesetzt werden kann.

Bei dem als Zuschlagstoff Z vorgesehenen Hektorit handelt es sich um ein natürlich vorkommendes Silikat-Mineral, welches in Form von Plättchenstapel-Aggregaten in nur wenigen Fundstätten vorkommt. Hektorit ist deutlich feinteiliger als der Bentonit, wobei bei mineralisch und kristallographisch weitestgehend identischem Aufbau die Plättchenstärke 1 nm und der Durchmesser ca. 500 nm beträgt und diese Plättchen flächig miteinander verbunden sind. Der optimale Effekt dieses Additives kann sich in den Anwendungen nur dann entfalten, wenn eine Zerteilung der Stapel in Einzelplättchen erreicht wird. Diese Plättchenstapel verhalten sich anschaulich wie Glasplatten, die durch definierte Scherkräfte des erfindungsgemäßen Knet-Misch-Aggregates 1 so gegeneinander verschoben werden, daß eine besonders schonende Trennung in unbeschädigte Einzelplättchen erfolgt und diese optimal verteilt werden. Mit dem erfindungsgemäßen Verfahrensablauf (Phase A, C, D) können überraschend die hohen Adhäsionskräfte im Hektorit und Bentonit überwunden werden, so daß bei wesentlicher Qualitätserhöhung des Endproduktes ein geringerer Materialeinsatz möglich ist.

Bei bisher bekannten Dispergierungen wird das Hektorit lediglich in aufwendigen Perl- und Kugelmühlen, Walzenstühlen o. dgl. Aggregaten verarbeitet, wobei auch der Einsatz von Homogenisatoren vorgesehen ist. Dabei wird ein Roh-Hektorit enthaltendes Lösemittelgemisch beispielsweise über eine Düse auf eine Hartmetallplatte beschleunigt, so daß in dieser ersten Verfahrensstufe ein relativ grobes Hektorit-Material erreicht wird und dieses unter Zusatz eines chemischen Aktivators in einem weiteren Mahldurchgang feiner zu dispergieren ist. Damit wurden bisher hohe Energiedichten auf ein relativ weiches Material eingetragen, so daß dieses einen vergleichsweise großen Anteil an zerstörten Plättchen aufweist. Bei diesen Produkten wird nach einem abschließenden Dispergierdurchlauf ein Endstadium erreicht, an das sich ein ca. 2-wöchiger Aktivierungs- und Reifeprozeß durch entsprechende Lagerung des Produkts anschließt. Erst danach kann am Ende aufwendiger und zeitintensiver Arbeitsschritte ein Gelkonzentrat als Lackrohstoff bereitgestellt werden.

Mit dem erfindungsgemäßen Verfahren und der Vorrichtung werden bisher sehr aufwendige Vorgänge der Herstellung von Lackrohstoffen auf einen in kürzerer Zeit durchführbaren Ablauf verkürzt und dabei mit geringerem Materialeinsatz ein Dispergier-Endergebnis höherer Qualität erreicht.

Das erfindungsgemäße Knet-Dispergier-Verfahren führt zu einer deutlichen Verbesserung des Hektorit-Aufschlusses, der als Festkörper-Konzentration im Lackrohstoff 1 zur Verfügung steht. Zur Demonstration der Dispergierunterschiede wurden jeweilige 3 %-ige NC-Lacke mit identischen Komponentenanteilen hergestellt, so daß auf der Basis von erfindungsgemäßen Hektorit-Chips und eines am Markt verfügbaren Gelkonzentrates ein direkter Vergleich der Eigenschaften möglich ist. Die hergestellten Lack-Lösungen wurden auf einer Aufstrichkarte in Form von identischen Auftragsschichten appliziert. Danach wurden unter dem Rasterelektronenmikroskop (REM) jeweilige Aufnahmen des entstandenen Schichtaufbaus senkrecht zur Beschichtungsebene angefertigt. Die Darstellung gemäß Fig. 7 zeigt eine aus dem Gelkonzentrat resultierende Schicht P, die eine niedrigere, uneinheitliche Beschichtung mit einer Dicke H bildet. Dabei sind unzerteilte Agglomerate durch die auseinanderfließende, offensichtlich niedrigviskosere Lacklösung freigelegt worden. Diese Schicht P sorgt durch diffuse Lichtstreuung für einen deutlich verringerten Glanz.

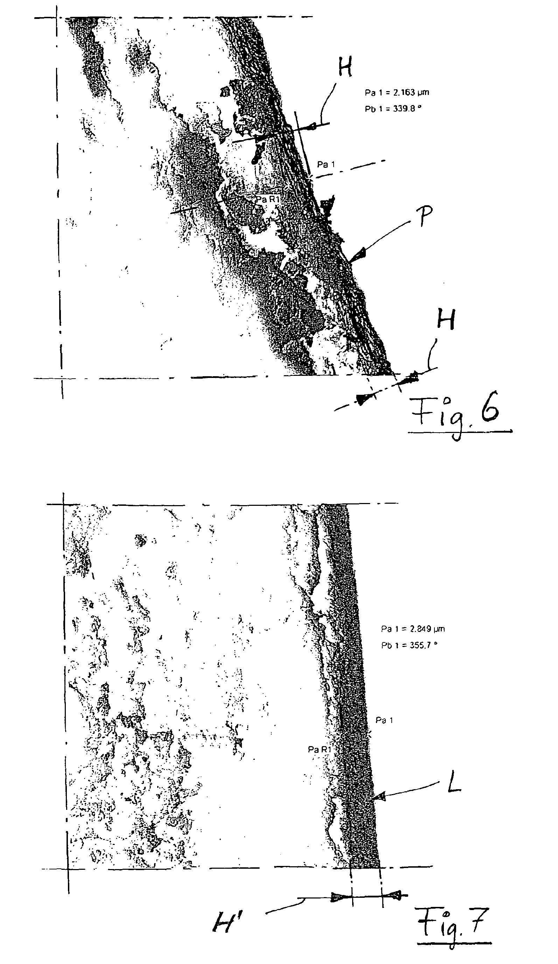

In Fig. 7 ist die Beschichtung L mit einem Lack aus erfindungsgemäßem Lackrohstoff zu sehen. Eine wesentlich höhere und gleichmäßigere Schichtstärke H' legt eine deutlich höhere Viskosität und Thixotropie des verwendeten Lackrohstoffes nahe. Der geradlinige Oberflächenverlauf läßt ebenso einen höheren Glanz erwarten. Dieser Glanz der Hektorit-Chip-Beschichtung L ist gemessen auch tatsächlich deutlich höher als mit dem Stand der Technik erreicht werden konnte (Fig. 6).

In Rezepturvariationen zeigte sich überraschenderweise, daß höhere Konzentrationen an Hektorit-Chip-Ausgangsmaterial in der Lackbeschichtung, entgegen der Erwartung, sogar einen noch höheren Glanz erreichen ließen. In der Anschauung hat man sich ein mit den Plättchen durch Kantenverknüpfung (über Wasserstoffbrücken) aufgebautes dreidimensionales Kartenhaus-Gerüst vorzustellen. Dieses reicht in höherer Konzentration bis kurz unter die Oberfläche der Schicht L und führt zu einer Stabilisierung des Glanzes auf einem hohen Niveau. Im Falle der vorgeschlagenen Pigmentierungen werden die Pigmentpartikel in diesen Kartenhaus-Zellen eingelagert und so an einer Sedimentation gehindert. Durch diese Anwesenheit von Mineralplättchen in Oberflächennähe ist darüber hinaus auch mit einer höheren Kratzbeständigkeit der erzeugten Lackschicht L zu rechnen.

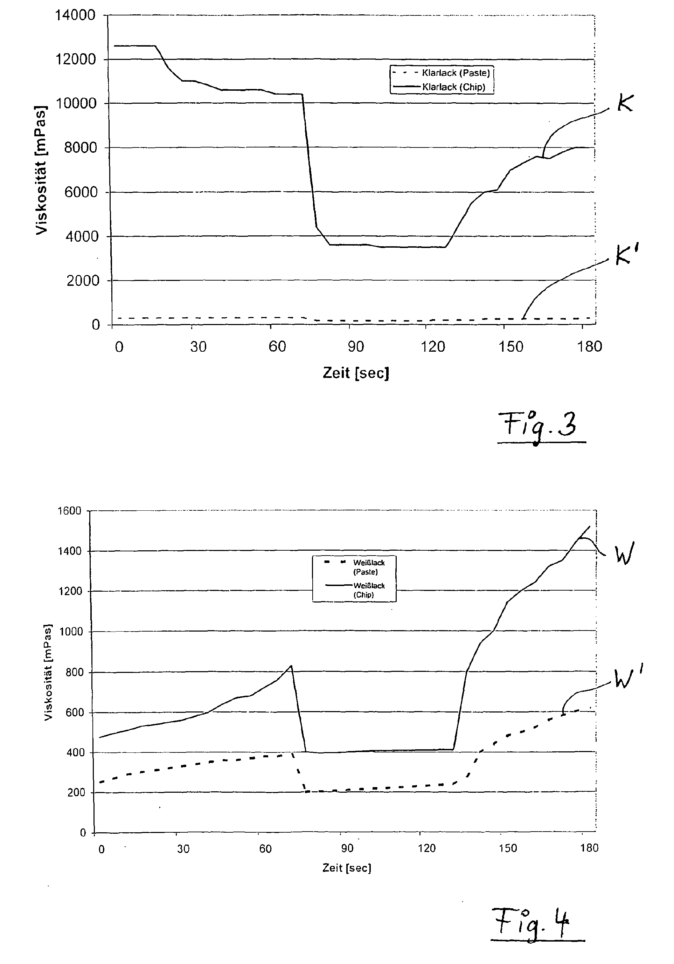

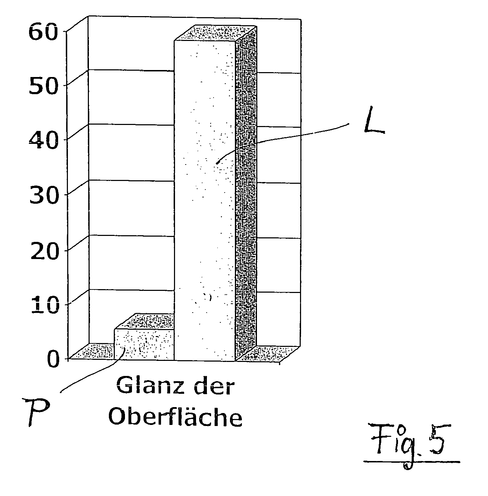

In Vergleichsversuchen wurde die Viskosität der für die REM-Aufnahmen verwendeten Lacklösungen gemessen. In einem Diagramm gemäß Fig. 3 sind auf der Ordinaten (y) die Viskosität und auf der Abszisse (x) der Messzeitverlauf aufgetragen. Bei Verwendung der erfindungsgemäßen Hektorit-Chips ergibt sich eine die höchste Viskosität belegende Meßkurve K für eine Klarlack-Formulierung. Die Anwendung eines bekannten Gelkonzentrates führt zu der untersten Diagrammkurve K'. Die Kurvenverläufe gemäß Fig. 4 zeigen Versuchs-Chip-Produktionen für einen Weißlack W erfindungsgemäßer Zusammensetzung und mit W' ist eine Kurve für eine Weißlackformulierung gemäß dem Stand der Technik gezeigt. Bei sämtlichen Messungen gemäß Fig. 3 und 4 wurden die Vergleichs-Lösungen zunächst in einem Ruhezustand gemessen. Dann wurden die Lösungen mit einem Rührgerät für kurze Zeit in Bewegung versetzt und dabei gemessen. Zum Abschluß wurde die gerührte Lösung nach Eintritt in die Ruhephase nochmals geprüft.

Der extrem starke Abfall der Hektorit-Chip-Kurven K bzw. W bei Rührereinsatz (Fig. 3 und 4, linke Seite) und der schnelle Anstieg im nachfolgenden Ruhezustand (Fig. 3 und 4, rechte Seite) belegt einen außerordentlich hohen Thixotropieeffekt dieses neuen Lackes. Mit dieser vorteilhaften Wirkung des erfindungsgemäßen Produkts wird beim Lackauftrag ein Auseinanderlaufen der Beschichtung und damit die Bildung der berüchtigten ,Nasen' verhindert. Außerdem wurde festgestellt, daß im Ruhezustand fertig präparierte Druck- und Lackfarben durch die verbesserten thixotropen Eigenschaften auch das Sedimentieren der im Lackrohstoff 1 als Pigmente enthaltenen Zuschlagstoffe Z vermieden ist. Der nachgewiesen starke Viskositätsabfall ist auch eine wesentliche Voraussetzung für verbesserte Verarbeitungseigenschaften von Lacken, die nach Formulierung mit dem Lackrohstoff 1 eine gute Verspritzbarkeit aufweisen.

### Beispiel 1

In ein auf ca. 70°C vorgeheiztes Knet-Misch-Aggregat 2 wird 277 kg mittelviskoses 35 % angefeuchtetes Cellulosenitrat (NC) zusammen mit 7 kg Ethylacetat und 45 kg Acetyltributylcitrat (ATBC) eingefüllt und innerhalb von 15 Minuten intensiv durchmischt. In dieses Gemenge werden nun innerhalb von weiteren 15 Minuten portionsweise 75 kg feinteiliger Hektorit (Bentone 27V) als Zuschlagstoff Z eingetragen und die Mischung M zu einem niedrigviskosen Teig verarbeitet. Die Beheizung wird nun beendet und die Mischung in den folgenden 120 Minuten mittels des Knetwerkes 4 sehr intensiv geschert. Das dabei erreichte hohe Viskositätsniveau wird dabei durch kontrollierten portionsweisen Zusatz von weiteren 63 kg Ethylacetat konstant gehalten. Der erzeugte hochviskose Teig wird als Zwischenprodukt 1' auf einer Scherwalze 8 (Fig. 2) weiterverarbeitet und in eine pelletierte Teilchenform überführt. Nach dem Trocknen (Fig. 1, Phase E') bei ca. 60°C resultiert als festes Produkt ein NC-Lackrohstoff 1, der eine Restfeuchte von ca. 0,5 % Ethylacetat aufweist.

Dieser NC-Lackrohstoff 1 wird eingesetzt, um die nachfolgend angegebenen Lack-Rezepturbeispiele I und II herzustellen. Zu Vergleichszwecken mit gemäß Stand der Technik bekannten Rezepturen wird ein im wesentlichen gleicher Lack auf Basis einer Bentone 27V-Gelpaste hergestellt. Diese Gelpaste wurde nach einem Homogenisator-Hochdruckdispergierverfahren hergestellt und enthält damit ebenfalls das Schichtsilikat (Hektorit), das in dem erfindungsgemäßen Chip-Lackrohstoff eingeknetet ist.

### Rezeptur I (Nagellack-Klarlackrezeptur)

| | |
|---|---|
| 21,8 Gew.-% | Ethylacetat |
| 31,0 Gew.-% | Butylacetat |
| 7,2 Gew.-% | Isopropanol |
| 16,1 Gew.-% | mittelviskose Nitrocellulose (trocken) |
| 12,5 Gew.-% | Aldehydbindemittel |
| 6,3 Gew.-% | Acetyltributylcitrat |
| 2,5 Gew.-% | Kampfer |
| 0,2 Gew.-% | Zitronensäure |
| 0,2 Gew.-% | Maleinsäure |
| 0,2 Gew.-% | Phosporsäure |
| 2,0 Gew.-% | Bentone 27V |

### Rezeptur II (Nagellack-Weißlackrezeptur)

| | |
|---|---|
| 17,4 Gew.-% | Ethylacetat |
| 24,8 Gew.-% | Butylacetat |
| 5,8 Gew.-% | Isopropanol |
| 13,0 Gew.-% | mittelviskose Nitrocellulose (trocken) |
| 10,0 Gew.-% | Aldehydbindemittel |
| 5,0 Gew.-% | Acetyltributylcitrat |
| 2,0 Gew.-% | Kampfer |
| 18,55 Gew.-% | Titandioxid |
| 0,15 Gew.-% | Zitronensäure |
| 0,15 Gew.-% | Maleinsäure |
| 0,15 Gew.-% | Phosphorsäure |
| 1,50 Gew.-% | Bentone 27V |

### Beispiel 2

In ein auf ca. 70°C vorgeheiztes Knet-Misch-Aggregat 2 wird 139 kg mittelviskoses 35 % angefeuchtetes Cellulosenitrat (NC) zusammen mit 6 kg Ethylacetat innerhalb von 15 Minuten intensiv vermischt. In dieses Gemenge werden nun innerhalb von weiteren 15 Minuten portionsweise 210 kg Iriodin-123 als Zuschlagstoff Z eingetragen und zu einem niedrigviskosen Teig verarbeitet. Die externe Heizung wird eingestellt und das Material als den Zuschlagstoff Z enthaltende Mischung M weitere 30 Minuten sanft geschert. Der als Zwischenprodukt 1' erhaltene Teig wird über eine Glattwalze 8 weiterverarbeitet und in eine Chipform überführt. Nach dem Trocknen bei ca. 60°C resultiert der Lackrohstoff 1 als ein festes Produkt, das eine Restfeuchte von ca. 0,5 % Ethylacetat aufweist.

Aus diesem Lackrohstoff wird zu weiteren Vergleichs-Untersuchungen ein Lack hergestellt. Hierzu wird eine 40 %-ige Lösung in einem Lösungsmittelgemisch-AL (10 Gew.-% Ethylacetat, 70 Gew.-% Ethanol und 20 Gew.-% Methoxy-2-propanol) nach Rezepturbeispiel III hergestellt. Zu den mit dem Stand der Technik vorgesehenen Vergleichszwecken wird die gleiche Rezeptur mit einem Skandex-Dispergieraggregat auf Basis der gleichen Ausgangskomponenten (Iriodin 123, mittelviskose Nitrocellulose) hergestellt.

### Rezeptur III (Perlglanz-Rezeptur)

| | |
|---|---|
| 6,0 Gew.-% | Ethylacetat |
| 42,0 Gew.-% | Ethanol |
| 12,0 Gew.-% | Methoxy-2-propanol |
| 8,0 Gew.-% | mittelviskose Nitrocellulose (trocken) |
| 4,0 Gew.-% | Acetyltributylcitrat |
| 28,0 Gew.-% | Iriodin 123 |

Von beiden Testkonzentraten (gemäß Beispiel 1 und 2) wurden vier NC-Lacklösungen hergestellt, die das unterschiedlich dispergierte Schichtsilikat (Chip, Gelpaste) in Konzentrationen von jeweils 1,5 % und 3,0 % enthielten. Mit einem Handcoator wurden von den Lacklösungen auf einem Fotoglanzkarton jeweilige Aufstriche mit einer Naßschichtstärke von 20 p angefertigt. Die getrockneten Beschichtungen wurden anschließend mit einem BYK-Gardner-Haze-Gloss bei einem Glanzwinkel von 60° vermessen. Es ergaben sich die in Fig. 5 und Tabelle 1 wiedergegebenen Glanzwerte (T = Paste, Gelpaste gemäß Stand der Technik; L = erfindungsgemäßer Lackrohstoff; Chip).

**Tabelle 1**

| Typ | Konzentration | Glanzwert |
|---|---|---|
| Paste | 1,5 Gew.-% | 7,3 % |
| Paste (T) | 3,0 Gew.-% | 5,6 % |
| Chip | 1,5 Gew.-% | 50,6 % |
| Chip (L) | 3,0 Gew.-% | 58,7 % |

Der Einsatz des erfindungsgemäßen Lackrohstoffes 1 als Chipmasse L führt gegenüber dem Vergleichsmaterial, hergestellt nach technischem Stand (Gelkonzentrat = Paste, T), zu einem deutlich verbesserten Glanzwert. Überraschend ist außerdem, daß bei Erhöhung der Schichtsilikat-Konzentration im Lackrohstoff L (Chipmasse) entgegen der zu erwartenden Verschlechterung (siehe Beispiel, Paste T), eine weitere Verbesserung des Glanzwertes um ca. 16 % festzustellen ist. Dies ist darauf zurückzuführen, daß die beim erfindungsgemäßen Lackrohstoff 1 (Chipmaterial L) optimal dispergierten Silikatplättchen eine ideal planparallele Lage zueinander einnehmen und dabei im Abstand zum Untergrund besonders nahe zur Oberfläche innerhalb der Lack-Beschichtung (Fig. 6) angeordnet sind. Neben einer sehr guten Glanzausbildung führt diese optimale Lage des Zuschlagstoffes Z auch zu einer Erhöhung der Kratzbeständigkeit (durch Messwerte aus der Pendelhärtenbestimmung nachweisbar). Beide Eigenschaften tragen zu einer deutlichen Verbesserung der Gebrauchseigenschaften von Lackbeschichtungen auf der Basis des Lackrohstoffes 1 bei.

Es ergibt sich damit, daß die Glanzwerte der Aufstriche mit nach dem erfindungsgemäßen Verfahren dispergiertem Hektorit-Zuschlagstoff Z jeweils im direkten Vergleich zum Stand der Technik eine ca. 10-fache Verbesserung aufweist. Auffällig ist, daß der 3,0 %-hektorithaltige Probe-Aufstrich gegenüber dem 1,5 %-hektorithaltigen Probe-Aufstrich auch einen erkennbar höheren Glanzgrad zeigt.

Die Probe-Aufstriche T und L (Tabelle 1) mit den Schichtsilikatkonzentrationen von jeweils 3 Gew.-% wurden mit einem Rasterelektronenmikroskop (REM) untersucht. Die Aufnahmen gemäß Fig. 6 und 7 zeigen die senkrecht zur Aufstrichrichtung angefertigten Querschnitte der entstandenen Lackschichten. Der Lack mit Verwendung des Pastenmaterials P gemäß Stand der Technik hat bei gleichen Bedingungen zu einer Klarlackschicht mit durchbrochenem Oberflächenverlauf (-> schlechtes Glanzverhalten) und einer mittleren Auftragsstärke H von ca. 2,16 µ (-> unvollständige Dispergierung der Silikatagglomerate zu Einzelplättchen) mit einem heterogenen, groblaminaren Schichtaufbau geführt. Die REM-Aufnahme gemäß Fig. 7 zeigt die Anwendung eines Lackes L auf Basis des erfindungsgemäßen Chip-Rohstoffes 1. Dabei wird eine Klarlackschicht erreicht, die einen durchgehend geradlinigen Oberflächenverlauf, eine vergleichsweise höhere mittlere Auftragsstärke H' von ca. 2,85 µ, gegenüber dem Pastenmaterial und einen homogenen feinlamillaren Schichtaufbau (-> feindispergierte Einzelplättchen) aufweist.

In weiteren Vergleichstest wurden rheologische Untersuchungen der Beispielrezepturen I und II durchgeführt. Es wurden jeweils ein Klarlack (Rezeptur 1) und ein Weißlack (Rezeptur II) unter Verwendung des Chip-Rohstoffes 1 und des Gelpräparates (Stand der Technik) hergestellt. Diese Lösungen wurden mit dem Rheometer Physikca MCR 300 (Paar Physica) unter Verwendung der Cone-Plate-Meßgeometrie untersucht. Die Proben wurden im Verlauf je einer Minute bei unterschiedlichen Scherraten (5 U/s, 50 U/s, 5 U/s) vermessen. Die Weißlacke I und II wurden unter Zugabe einer gleichen Menge an Polyethylencarbonat vollständig aktiviert. Aus den gemessenen Viskositätswerten wurde der Thixotropie-Index berechnet (siehe Tabelle 2):

**Tabelle 2**

| Probentyp | Viskosität (cPs) | | | Thixotropie-Index |
|---|---|---|---|---|
| | 5 U/s | 50 U/s | 5 U/s | |
| Pasten-Klarlack (P) | 184 | 157 | 222 | 141,00 |
| Chip-Klarlack (L) | 10525 | 3552 | 7509 | 2,11 |
| Pasten-Weißlack (P) | 416 | 255 | 635 | 2,49 |
| Chip-Weißlack (L) | 830 | 412 | 1607 | 3,90 |

Das erfindungsgemäße Chip-Produkt L zeigt sich in allen Belangen den bekannten Gel-Präparaten P überlegen. Die erreichbaren Viskositätswerte liegen sowohl im nicht aktivierten Zustand als auch nach der Aktivierung bei dem erfindungsgemäßen Lackrohstoff deutlich höher. Damit zeigt sich, daß nicht nur eine sehr gute Dispergierung sondern auch ein sehr hoher Aktivierungsgrad der als Zuschlagstoff Z dispergierten Plättchen im Lackrohstoff 1 erreicht wird. Bei der Herstellung von Endprodukten aus dem Lackrohstoff 1 ist es möglich, durch geringere Mengen dieses Chip-Materials einen gleichhohen Aktivierungseffekt zu erreichen, so daß bei einer vorteilhaften Materialersparnis gleichzeitig ein größerer Rezeptierungsspielraum bei der Endproduktherstellung erreicht wird. Der bereits hohe Aktivierungsgrad der Produkte läßt sich durch Zusatz von polaren Aktivatoren zum Dispergierprozeß noch weiter optimieren. Auch dabei sind für den erfindungsgemäßen Lackrohstoff 1 geringere Mengen dieser Zusätze erforderlich, da diese konzentrierter und direkter auf die Zuschlagstoffe Z in Form der Silikatplättchen einwirken können. Der bisher übliche Einsatz von Aktivatoren in verdünnten Lösungen kann damit insgesamt entbehrlich sein.

## Patentansprüche

1. Verfahren zur Herstellung eines insbesondere Cellulosenitrat (NC) als thermoplastisches Bindemittel enthaltenden Rohstoffes für Lacke, Farben o. dgl. Produkte, wobei in diesem unter Zusatz von Lösungsmittel und Weichmacher ein eine plättchenförmige Struktur aufweisender Zuschlagstoff (Z) verteilt wird, **dadurch gekennzeichnet, daß** das thermoplastische Bindemittel unter Zusatz von Lösungsmittel und Weichmacher oder ohne diesen zu einem niedrigviskosen Teig auf 40°C bis 70°C aufgeheizt, direkt mit dem plättchenförmigen Zuschlagstoff (Z) gemischt, bei weiterem Lösungsmittelzusatz diese endproduktnahe Mischung (M) in eine bei 60°C bis 110°C plastifizierte teigige Konsistenz überführt und diese abhängig vom Zuschlagstoff (Z) hoch- oder niedrigviskose Mischung (M) geknetet wird, derart, daß nach einer definierte Scherkräfte in die Mischung (M) eintragenden Knetphase (A) ein den Zuschlagstoff (Z) in dispergierter Form enthaltender Lackrohstoff (1) bereitgestellt wird.

2. Verfahren nach Anspruch 1, **dadurch gekennzeichnet, daß** bei Verwendung feinstteiliger Schichtsilikate als Zuschlagstoff (Z) während der Knetphase (A) eine portionsweise Zugabe von Lösungsmittel erfolgt, dabei maximale Scherkräfte in die einen homogenen Teig bildende Mischung (M) eingetragen werden und dabei gleichzeitig eine Viskositätskontrolle (6) durchgeführt wird.

3. Verfahren nach Anspruch 1, **dadurch gekennzeichnet, daß** bei Verwendung von beschichteten Effektpigmenten als Zuschlagstoff (Z) und portionsweiser Zugabe von Lösungsmittel während der Knetphase (A) ein im Vergleich mit feinstteiligen Schichtsilikaten geringerer Scherkrafteintrag in die den homogenen Teig bildende Mischung (M) erfolgt, wobei diese gleichzeitig durch eine Viskositätskontrolle (6) überwacht wird.

4. Verfahren nach einem der Ansprüche 1 bis 3, **dadurch gekennzeichnet, daß** der mit Schichtsilikat (Z) plastifizierten Mischung (M) zumindest während der Knetphase (A) zumindest eine Aktivierungskomponente in Form von Polyethylcarbonat, Zitronensäure, Phosphorsäure, Maleinsäure o. dgl. zugegeben wird.

5. Verfahren nach einem der Ansprüche 1 bis 4, **dadurch gekennzeichnet, daß** zumindest während der Knetphase (A) ein oder mehrere Weichmacher in Form von Acetyltriethylcitrat (ATC), Acetyltributylcitrat (ATBC), Phtalate, Sebacate, Adipate, Phosphate o. dgl. zugesetzt werden.

6. Verfahren nach einem der Ansprüche 1 bis 5, **dadurch gekennzeichnet, daß** der Mischung (M) jeweils Lösungsmittel in Form von Estern (z. B. Methylacetat, Ethylacetat, Isopropylacetat), Ketonen (z. B. Aceton, MEK, MIBK), Alkoholen (z. B. Ethanol, Propanol, Butanol) und/oder Wasser zugesetzt werden/wird.

7. Verfahren nach einem der Ansprüche 1 bis 6, **dadurch gekennzeichnet, daß** der Anteil an zuzugebendem Lösungsmittel entsprechend dem Bindemittel, der Art des Zuschlagstoffes (Z), dessen Anteil an der Gesamtmenge der Mischung (M), deren Temperatur und/oder der erreichten Viskosität bemessen wird.

8. Verfahren nach einem der Ansprüche 1 bis 7, **dadurch gekennzeichnet, daß** der Lackrohstoff als fertiggeknetetes Zwischenprodukt (1') nach einer Reduktion des Lösemittelgehaltes durch einen zumindest einstufigen Walz-, Pelletier- und/oder Preßvorgang (C, D) enddispergiert, getrocknet und zu Strukturteilen (7) in Form von Pellets, Granulat, Chips oder Pulver zerkleinert wird.

9. Verfahren nach einem der Ansprüche 1 bis 8, **dadurch gekennzeichnet, daß** das mit einem Effektpigment fertiggeknetete Zwischenprodukt (1') beim nachfolgenden Walz-, Pelletier- und/oder Preßvorgang (C, D) jeweiligen den in der Knetphase (A) wirksamen Kräften weitgehend entsprechenden Scherkräften ausgesetzt wird und damit der koloristische Zustand und/oder die Glanzeigenschaften des Lackrohstoffes (1) beeinflußt werden.

10. Verfahren nach einem der Ansprüche 1 bis 9, **dadurch gekennzeichnet, daß** nach einer Kontrolle des Dispergierzustandes des Zuschlagstoffes (Z) im Lackrohstoff (1') dessen Endtrocknung (D) durchgeführt und dieser in die Strukturteile (7) zerkleinert wird.

11. Verfahren nach einem der Ansprüche 1 bis 10, **dadurch gekennzeichnet, daß** der als Zwischenprodukt (1') bearbeitete Lackrohstoff mehrfach geknetet und/oder einem Preßvorgang unterworfen wird.

12. Verfahren nach einem der Ansprüche 1 bis 11, **dadurch gekennzeichnet, daß** an Stelle des NC-Bindemittels jeweils Celluloseacetopropionate, Celluloseacetobutyrate, Polyvinylbutyrate, Polyvinylchlorid-Mischpolymerisate, Acrylate, Polyester, Ethylcellulosen oder Gemische mehrerer dieser Stoffe mit dem plättchenförmigen Zuschlagstoff (Z) geknetet werden.

13. Vorrichtung zur Durchführung des Verfahrens gemäß einem der Ansprüche 1 bis 12, **dadurch gekennzeichnet, daß** zur Verteilung der Mischungsbestandteile ein an sich bekanntes, zur Dispergierung eines plättchenförmigen Zuschlagstoffes (Z) steuerbares und eine neben Weichmacher sowie Lösungsmittel insbesondere Cellulosenitrat (NC) als Bindemittel enthaltende Mischung (M) sicher verarbeitendes Knet-Misch-Aggregat (2) vorgesehen ist.

14. Vorrichtung nach Anspruch 13, **dadurch gekennzeichnet, daß** mit dem auf einen definierten Scherkrafteintrag einstellbaren Knet-Misch-Aggregat (2) eine Feinverteilung des Zuschlagstoffes (Z) in einem festen Zustand des NC-Bindemittels durchführbar ist.

15. Vorrichtung nach Anspruch 13 oder 14, **dadurch gekennzeichnet, daß** das Knet-Misch-Aggregat (2) eine diskontinuierlich arbeitende Dispergiervorrichtung bildet.

16. Vorrichtung nach Anspruch 13 oder 14, **dadurch gekennzeichnet, daß** das Knet-Misch-Aggregat (2) in Form einer kontinuierlich arbeitenden Baueinheit vorgesehen ist.

17. Vorrichtung nach einem der Ansprüche 13 bis 16, **dadurch gekennzeichnet, daß** das in Form eines Trogkneters (3), Innenmischers o. dgl. vorgesehene Knet-Misch-Aggregat (2) eine als Knet- und Mischwerk wirksame Baugruppe (5, 5') aufweist, mit der die in den Bestandteilen variabel zusammensetzbare Mischung (M) mit Zuschlagstoff (Z) sowie NC-Bindemittel durch Preß- und Zugkräfte beeinflußbar und dabei das jeweilige Maß der Viskosität kontrollierbar ist.

18. Vorrichtung nach einem der Ansprüche 13 bis 17, **dadurch gekennzeichnet, daß** das Knet-Misch-Aggregat (2) beheizbar und kühlbar ist, derart, daß aus dem Aggregat (2) das Lösungsmittel über eine Öffnung (12) o. dgl. absaugbar und danach das Zwischenprodukt (1') mit vollständig dispergiertem Zuschlagstoff (Z) granulierbar ist.

19. Lackrohstoff in Form einer einen Zuschlagstoff enthaltenden Farb- oder Lackpräparation, hergestellt mit einem Verfahren nach einem der Ansprüche 1 bis 12 bzw. einer Vorrichtung gemäß einem der Ansprüche 13 bis 18, **dadurch gekennzeichnet, daß** der Lackrohstoff 1 % bis 70% eines plastifizierbaren Bindemittels, vorzugsweise Cellulosenitrat (NC), 1 % bis 40% Weichmacher und 1 % bis 85% eines plättchenförmigen Zuschlagstoffes (Z) enthält.

20. Lackrohstoff nach Anspruch 19, **dadurch gekennzeichnet, daß** als Zuschlagstoff (Z) Schichtsilikate, plättchenförmige Füllstoffe und/oder Effektpigmente vorgesehen sind.

21. Lackrohstoff nach Anspruch 19 oder 20, **dadurch gekennzeichnet, daß** der Zuschlagstoff (Z), vorzugsweise feinteiliges Schichtsilikat, mit einem Anteil von weniger als 40% eingearbeitet ist.

22. Verwendung eines einen dispergierten Zuschlagstoff enthaltenden Lackrohstoffes, hergestellt nach einem Verfahren bzw. einer Vorrichtung gemäß einem oder mehreren der Ansprüche 1 bis 21, zu einer Formulierung von Druckfarben, Lacken, Nagellacken, Lack-Aerosole o. dgl. Endprodukten.
